# EUROPÄISCHE PATENTANMELDUNG

(11) **EP 3 714 926 A1**
(43) Veröffentlichungstag der Anmeldung: **30.09.2020**
(21) Anmeldenummer: 19165793.1
(22) Anmeldetag: 28.03.2019
(51) Int. Cl.: A61M 5/30, A61B 17/00

(54) **INSTRUMENT, INSTRUMENTENKOPF, APPLIKATIONSSYSTEM UND VERFAHREN**

(71) Anmelder: Erbe Elektromedizin GmbH, 72072 Tübingen (DE)
(72) Erfinder: Enderle, Markus D., 72070 Tübingen (DE); Fech, Andreas, 72070 Tübingen (DE); Brodbeck, Achim, 72555 Metzingen (DE)
(74) Vertreter: Rüger Abel Patentanwälte PartGmbB

(57) **Zusammenfassung**

Um eine hohe Wirksamkeit von einer in Gewebe eines Patienten eingebrachten Substanz zu gewährleisten, ist erfindungsgemäß ein Instrument (10) mit einer ersten Leitung (11) zum Ausgeben eines ersten Fluids (16) aus einer ersten Öffnung (12) der ersten Leitung (11) in Axialrichtung (A) in eine Nachbeschleunigungszone (19) und mit einer zweiten Leitung (14) zum Leiten von zweitem Fluid (17) in die Nachbeschleunigungszone (19) in Axialrichtung (A), so dass Wirkstoffbestandteile des zweiten Fluids (17) in Folge des Strömens in die Nachbeschleunigungszone (19) von dem in die Nachbeschleunigungszone (19) eintretenden ersten Fluid (16) in Axialrichtung (A) nachbeschleunigt werden. Es wird ein koaxialer Aufbau der ersten und zweiten Leitungen (11, 14) bevorzugt, auf Grund dessen um die erste Öffnung (12) ein in Axialrichtung (A) stromabwärts reichender Mantelstrahl (21) aus zweitem Fluid (17) um den zentralen Arbeitsstrahl (20) erzeugt werden kann. Zudem ist ein Kopf (24) für ein erfindungsgemäßes Instrument (10) und ein Applikationssystem (100) mit einem erfindungsgemäßen Instrument (10) geschaffen.

## Beschreibung

Die Erfindung betrifft ein Instrument, insbesondere ein Instrument zum Applizieren einer Substanz in Gewebe, einen Instrumentenkopf, ein Applikationssystem sowie ein Verfahren.

In aus dem Stand der Technik bekannten Verfahren zum Injizieren eine Substanz in Gewebe kann eine Injektionskanüle gepaart mit einer Einmalspritze verwendet werden. Die Injektionskanüle (Kanüle) kann beispielsweise einen Durchmesser von bis zu 1,6 mm aufweisen. Bei der manuellen Applikation und Injektion einer Substanz mit einer Injektionsnadel bestehen ein erhöhtes Risiko einer mangelhaften Positionierung, sowie die Gefahr von Perforationen des Gewebes an unerwünschter Stelle. Zudem tritt eine vergleichsweise große Gewebetraumatisierung an der Einstichstelle auf Grund des großen Durchmessers der Kanüle (bis zu 1,6 mm). Außerdem führt ein großer Einstich- bzw. Öffnungskanal dazu, dass eine große Menge der eingebrachten Substanz wieder aus dem Gewebe austreten und so wertvolles Material verloren gehen kann. Neben dem Verlust kann dies auch zu vermehrter Ansammlung der Substanz in Gewebearealen außerhalb des Zielbereichs führen. Eine Schädigung eines Muskels auf Grund des Einstechens, was zu einer Vernarbung (Fibrose) des gesunden Muskelgewebes führen kann, ist ein weitere Nachteil, insbesondere wenn mit einer Nadel pro Einstich jeweils nur ein kleines Volumen an Substanz in einem räumlich stark eingeschränkten Bereich abgegeben werden kann, und deshalb beispielsweise für eine umlaufende zirkuläre Behandlung des Schließmuskels mehrere Einstiche erforderlich sind. Neben dem erhöhten Zeiteinsatz sinkt dadurch auch die Effektivität der Behandlung, da hier ein zusätzlicher Effekt in dem Muskel geschaffen wird, der im schlimmsten Fall die Regenerationswirkung der Therapie mittels der Substanz übersteigt. Dieser Aspekt gewinnt insbesondere in Anbetracht des relativ großen Kanülendurchmessers von bis zu 1,6 mm an Bedeutung.

In EP 3 040 036 A1 wird ein Ansatz zum nadellosen Eintragen der Zellen mittels eines Wasserstrahls vorgeschlagen. Dabei erlaubt das vorgeschlagene Instrument eine räumlich und zeitlich getrennte Zufuhr zweier Substanzen, so dass z.B. eine sequentielle Applikation der Zellen und eines Trägermediums möglich ist.

Die EP 3 040 101 A1 offenbart ein Pumpen- und Dosiersystem, das eine sequentielle Applikation mit unterschiedlich hohen Druckniveaus erlaubt.

EP 2 907 582 A1 offenbart eine Vorrichtung zum Versprühen von medizinischen Fluiden in überlappenden Sprühkegeln.

Bei der Applikation von Zellen können auf diese ausgeübte Scherkräfte die Zellen beschädigen. Von Vorteil wäre daher ein Konzept zur nadellosen Injektion einer Substanz, insbesondere von Wirkstoffen, insbesondere Zellen, bei welcher die Funktionstüchtigkeit der Wirkstoffe weitgehend erhalten bleibt, insbesondere die Beschädigung von Zellen, welche eingetragen werden sollen, weitgehend vermieden wird.

Diese Aufgabe wird mit einem Instrument gemäß Anspruch 1, einem Instrumentenkopf gemäß Anspruch 13, einem Applikationssystem gemäß Anspruch 14 und einem Verfahren gemäß Anspruch 16 gelöst:
Das erfindungsgemäße Instrument weist eine erste Leitung zum Ausgeben eines ersten Fluids aus einer ersten Öffnung der ersten Leitung in Axialrichtung in eine Nachbeschleunigungszone auf. Das Instrument weist zudem eine zweite Leitung zum Leiten von zweitem Fluid in die Nachbeschleunigungszone in Axialrichtung auf, so dass zumindest Bestandteile des zweiten Fluids infolge des Strömens in die Nachbeschleunigungszone von dem in die Nachbeschleunigungszone eintretenden ersten Fluid in Axialrichtung nachbeschleunigt werden. Das erste Fluid kann auch als Arbeitsfluid bezeichnet werden. Das zweite Fluid kann auch als Wirkstofffluid bezeichnet werden und Zellen enthalten, welche mittels des Instruments in Gewebe appliziert werden sollen.

Beim Eintreten in die Nachbeschleunigungszone hat das zweite Fluid, insbesondere dessen Bestandteile, welche nachbeschleunigt werden sollen, bereits eine Geschwindigkeitskomponente in Axialrichtung. Die Relativgeschwindigkeit in Axialrichtung zwischen dem ersten Fluid zum Nachbeschleunigen der Bestandteile des zweiten Fluids und den Bestandteilen ist deshalb kleiner als die Geschwindigkeit des ersten Fluids. Dadurch sind die Bestandteile, beispielsweise Zellen, geringeren Geschwindigkeiten, Drücken, Geschwindigkeits- und Druckunterschieden ausgesetzt und folglich auch geringeren Stoß- und Scherbelastungen, wodurch sie wesentlich schonender in das Gewebe transportiert werden können.

Weitere vorteilhafte Merkmale des erfindungsgemäßen Instruments, des erfindungsgemäßen Instrumentenkopfs, des erfindungsgemäßen Applikationssystems und des erfindungsgemäßen Verfahrens ergeben sich aus der folgenden Beschreibung.

Die zweite Leitung weist bevorzugt eine Vorbeschleunigungszone stromaufwärts der Nachbeschleunigungszone auf. Das Instrument ist bevorzugt dazu eingerichtet, das zweite Fluid in der Vorbeschleunigungszone in Axialrichtung vorzubeschleunigen. Die erste Öffnung ist an der Nachbeschleunigungszone angeordnet, so dass das aus der ersten Öffnung ausgegebene erste Fluid das vorbeschleunigte zweite Fluid in Axialrichtung nachbeschleunigt. Mittels der Vorbeschleunigungszone kann das zweite Fluid erst kurz vor der Nachbeschleunigungszone auf eine erhöhte Geschwindigkeit gebracht werden, um die Relativgeschwindigkeit zwischen dem ersten Fluid und dem zweiten Fluid am Anfang der Nachbeschleunigungszone zu reduzieren. Eine stromaufwärts der Nachbeschleunigungszone benachbart zum distalen Ende des Instruments angeordnete Vorbeschleunigungszone hat den Vorteil, dass das zweite Fluid bis zu der Vorbeschleunigungszone benachbart zum distalen Ende des Instruments mit niedriger Geschwindigkeit gefördert werden kann und das zweite Fluid erst in Nachbarschaft zum distalen Ende des Instruments, beispielsweise in einem Instrumentenkopf, vorbeschleunigt wird.

Die Vorbeschleunigungszone ist vorzugsweise von einem Düsenabschnitt der zweiten Leitung gebildet, in welchem sich die Strömungsquerschnittsfläche der zweiten Leitung in Strömungsrichtung (Axialrichtung) verjüngt. Das Verhältnis der Geschwindigkeit in einem Querschnitt zu der Geschwindigkeit in einem verjüngten Querschnitt ist bekanntermaßen proportional zum Verhältnis der verjüngten Querschnittsfläche zur Querschnittsfläche.

Eine Druckpulsation des zweiten Fluids in und/oder nach dem Düsenabschnitt wird weitgehend vermieden, wenn sich der Querschnitt in zumindest einem Unterabschnitt des Düsenabschnitts kontinuierlich verjüngt.

Bevorzugt umgibt die zweite Leitung die erste Leitung an der ersten Öffnung konzentrisch, um einen ringförmigen Mantelstrom an zweitem Fluid um das aus der ersten Öffnung austretende Fluid zu bilden. Dies fördert eine gleichmäßige Beladung der Oberfläche des Strahls aus erstem Fluid mit Bestandteilen aus dem zweiten Fluid.

Bevorzugt ist die zweite Leitung dazu eingerichtet, den Mantelstrom radial eng an die erste Leitung zu führen, so dass der Arbeitsstrahlstrahl, welcher aus der ersten Öffnung ausgegebene wird, Bestandteile des Mantelstroms in den Arbeitsstrahl einsaugt. Dieser Effekt wird auf Grund der zunächst höheren Geschwindigkeit des Arbeitsstrahls relativ zu dem Mantelstrom auf den Bernoulli-Effekt zurückgeführt.

Bevorzugt ist die Außenwand der zweiten Leitung in einem Abschnitt um die erste Öffnung elastisch. Dies führt zu einer Selbstzentrierung der zweiten Leitung um die erste Öffnung, um beispielsweise relativ grobe Toleranzen auszugleichen, innerhalb deren Toleranzen die zweite Leitung die erste Leitung an der ersten Öffnung konzentrisch umgibt.

Der Abschnitt kann von einem elastischen Element gebildet sein, welches auch den Düsenabschnitt vollständig oder teilweise bildet. Eine Selbstzentrierung kann daher im Düsenabschnitt erfolgen.

Das elastische Element ist bevorzugt stromaufwärts fluiddicht mit einem Rohrabschnitt der zweiten Leitung verbunden. Radial zwischen dem Rohrabschnitt und der ersten Leitung kann eine Zentriereinrichtung wirksam sein. Der Rohrabschnitt ist in Radialrichtung starr, unnachgiebig. Dies fördert letztendlich die Bildung eines ringförmigen Mantelstroms an zweitem Fluid um den Trägerstrom an erstem Fluid.

Der in Radialrichtung unnachgiebige Rohrabschnitt der zweiten Leitung umgibt vorzugsweise einen in Radialrichtung unnachgiebigen Rohrabschnitt der ersten Leitung, wobei die Zentriereinrichtung radial zwischen dem Rohrabschnitt der zweiten Leitung und dem Rohrabschnitt der ersten Leitung angeordnet sein kann, um den Rohrabschnitt der ersten Leitung und den Rohrabschnitt der zweiten Leitung konzentrisch auszurichten.

Bevorzugt ist die Nachbeschleunigungszone, in welcher auch der Eintrag von Bestandteilen des zweiten Fluids in den Trägerstrom stattfindet, innerhalb des Instruments ausgebildet. Das Instrument weist bevorzugt eine distale Öffnung auf, aus welcher der Trägerstrom mit Bestandteilen des zweiten Fluids stromabwärts der Nachbeschleunigungszone aus dem Instrument austritt.

Mit der distalen Öffnung des Instruments kann das Instrument beispielsweise auf das Gewebe aufgesetzt werden, in welches mittels des Instruments ein Injektionskanal nadellos mittels eines Pilotstrahls aus erstem Fluid eingebracht werden kann.

Die zweite Leitung endet vorzugsweise stromabwärts der ersten Öffnung. Anders gesagt umgibt die zweite Leitung vorzugsweise zumindest einen Abschnitt der Nachbeschleunigungszone. Dies führt dazu, dass der Strom aus zweitem Fluid in der Nachbeschleunigungszone in einer vorbestimmten Form bleibt und nicht übermäßig nach außen divergiert. Die zweite Leitung ist in dem über die erste Öffnung überstehenden Abschnitt vorzugsweise zylindrisch. Dies fördert die Übertragung von Bestandteilen des zweiten Fluids auf den Trägerstrom.

Die zweite Leitung endet bevorzugt stromaufwärts der distalen Öffnung des Instruments. Dies führt dazu, dass auch beim Aufsetzen des in Axialrichtung bevorzugt drucksteifen distalen Endabschnitts des Instruments mit der distalen Öffnung auf Gewebe das Gewebe nicht an das distale Ende der zweiten Leitung heranreicht und damit Druck auf die zweite Leitung ausübt und die zweite Leitung möglicherweise verformt, insbesondere, wenn diese ein elastisches Leitungselement am distalen Ende aufweist.

Das Instrument ist dazu eingerichtet, das erste Fluid und das zweite Fluid bis benachbart zum distalen Ende des Instruments heran nebeneinander in nebeneinander angeordneten Leitungsabschnitten, vorzugsweise parallel zueinander, zu führen und anschließend weiter in Richtung distalem Ende in koaxialen Leitungsabschnitten.

Bevorzugt ist die Vorbeschleunigungszone und/oder die Nachbeschleunigungszone in einem Kopf des Instruments angeordnet. Das zweite Fluid legt damit zwischen der Vorbeschleunigungszone und/oder der Nachbeschleunigungszone und dem distalen Ende des Instruments nur noch eine geringe Strecke zurück. Beschädigungen der Zellen durch Transport über eine längere Strecke bei höherer Geschwindigkeit werden damit vermieden.

Der Kopf des Instruments ist bevorzugt austauschbar.

Erfindungsgemäß wird auch ein Instrumentenkopf für ein Instrument angegeben. Dieser enthält vorzugsweise die Vorbeschleunigungszone und/oder die Nachbeschleunigungszone. Der Instrumentenkopf ist vorzugsweise an proximalen Leitungsabschnitten der ersten und zweiten Leitung austauschbar befestigbar.

Es wird zudem ein Applikationssystem mit einer beliebigen der Ausführungsformen des erfindungsgemäßen Instruments angegeben. Zusätzlich weist das Applikationssystem eine Versorgungseinrichtung auf, die mit der ersten Leitung und der zweiten Leitung in fluide Verbindung bringbar ist und dazu ausgebildet ist, das erste Fluid und das zweite Fluid in einer Abfolge von Förderintervallen zu befördern.

Bevorzugt weist die Versorgungseinrichtung eine Steuerung auf, welche das Applikationssystem derart steuert, dass innerhalb eines Applikationszeitinterfalls während eines ersten Arbeitsfluidförderintervalls das erste Fluid gefördert wird, so dass dieses an der ersten Öffnung eine erste Geschwindigkeit zum Bilden eines Kanals im Gewebe aufweist. Die Steuerung steuert das Applikationssystem zudem derart, dass während eines zweiten Arbeitsfluidförderintervalls das erste Fluid gefördert wird, so dass es an der ersten Öffnung eine reduzierte erste Geschwindigkeit aufweist, die kleiner als die erste Geschwindigkeit ist. Die Steuerung steuert das Applikationssystem zudem derart, dass zumindest abschnittsweise während des zweiten Arbeitsfluidförderintervalls das zweite Fluid gefördert wird, so dass das zweite Fluid eine zweite Geschwindigkeit an der ersten Öffnung aufweist, welche kleiner ist als die reduzierte erste Geschwindigkeit.

Das erfindungsgemäße Verfahren zum Applizieren von Wirkstoff, insbesondere Zellen, in Gewebe, weist den Schritt des nadellosen Öffnens eines Kanals in dem Gewebe mittels eines Arbeitsstrahls aus erstem Fluid ausgestoßen aus einer ersten Leitung aus einer ersten Öffnung eines Instruments auf. Das Instrument ist bevorzugt ein erfindungsgemäßes Instrument wie hierin beschrieben. In einem zweiten Schritt wird parallel oder koaxial zum Arbeitsstrahl ein Strahl aus zweitem Fluid aus einer zweiten Leitung des Instruments ausgegeben, welches zweite Fluid Bestandteile - Wirkstoff, insbesondere Zellen - aufweist, welche von dem Arbeitsstrahl aufgenommen und in den Kanal getragen werden. Die Bestandteile werden vorzugsweise durch Ansaugen von zweitem Fluid aus dem Strahl aus zweitem Fluid in den Arbeitsstrahl aufgenommen. Zwischen dem ersten Schritt und dem zweiten Schritt kann die Ausgabe des Arbeitsstrahls unterbrochen werden. Bevorzugt aber wird der Arbeitsstrahl für den zweiten Schritt nur abgeschwächt, insbesondere mit niedrigerer Geschwindigkeit ausgegeben als zur Öffnung des Kanals, ansonsten aber ununterbrochen über den ersten und den zweiten Schritt ausgegeben. Damit kann sichergestellt werden, dass der mit dem Arbeitsstrahl geschaffene Kanal von diesem offengehalten wird.

Weitere optionale vorteilhafte Merkmale und Ausführungsbeispiele ergeben sich aus der nachfolgenden Beschreibung sowie den Zeichnungen. Es zeigen:
Figur 1a - stark schematisiert ein Ausführungsbeispiel des erfindungsgemäßen Instruments in einer Teillängsschnittansicht,
Figur 1b - eine Ansichten eines Querschnitts des Ausführungsbeispiels gemäß Figur 1a zur Veranschaulichung eines Selbstzentriereffekts,
Figur 2 - ein weiteres Ausführungsbeispiel des erfindungsgemäßen Instruments in einer Längsschnittansicht,
Figur 3a - eine vergrößerte Ansicht eines in Figur 2 eingezeichneten Ausschnitts des Instruments gemäß des Ausführungsbeispiels nach Figur 2,
Figur 3b - eine Schnittansicht eines Bereich einer Schnittebene durch Figur 3a,
Figur 3c - ein Querschnitt des Instruments entlang der Schnittlinie B-B, eingezeichnet in Figur 3,
Figur 4 - eine Detailansicht eines Abschnitts, eingezeichnet in Figur 2,
Figur 5a - eine stark schematisierte Ansicht eines Ausführungsbeispiels des erfindungsgemäßen Applikationssystems,
Figur 5b - ein Ausschnitt aus Figur 5a,
Figur 6 - Diagramme zur Veranschaulichung eines zeitlichen Ablaufs einer Steuerung des Applikationssystems gemäß Figur 6a.

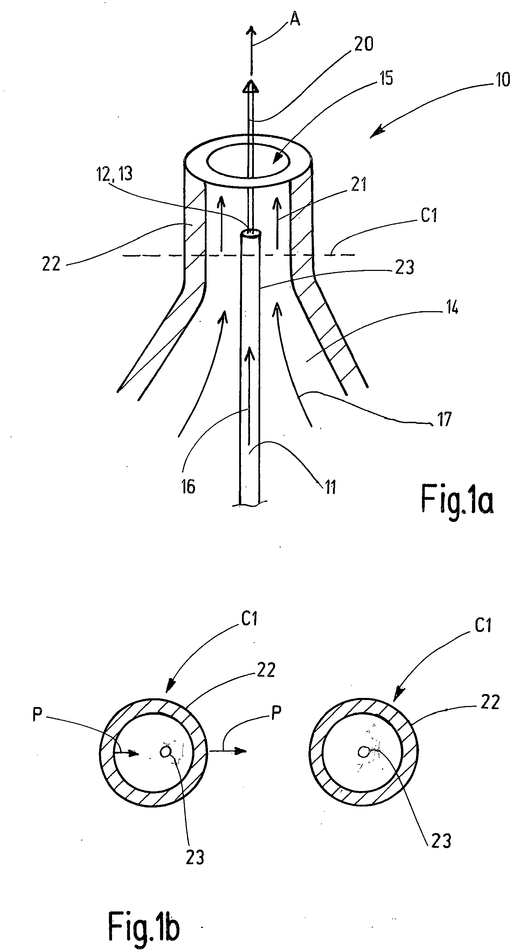

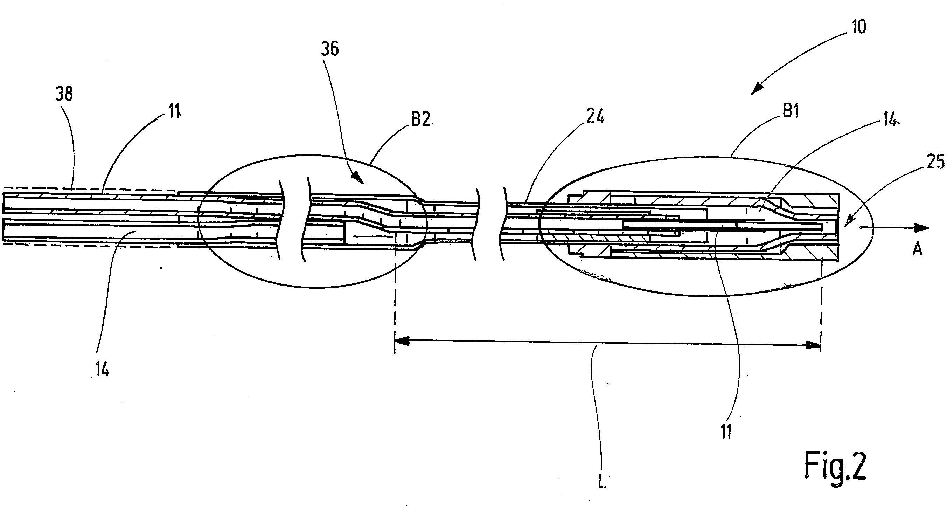

Figur 1a zeigt stark schematisiert ein erfindungsgemäßes Instrument 10 mit einer ersten Leitung 11 mit einer ersten Öffnung 12 an der Stirnseite 13 der ersten Leitung 11. Die erste Leitung 11 ist in einer zweiten Leitung 14 angeordnet. In dem Ausführungsbeispiel bildet die erste Leitung 11 eine Innenwand 15 der zweiten Leitung 14. Die erste Leitung 11 und die zweite Leitung 14 sind koaxial. Die erste Öffnung 12 ist stromaufwärts der Öffnung 15(zweite Öffnung) der zweiten Leitung angeordnet. Die erste Leitung 11 und die zweite Leitung 14 werden von dem ersten Fluid 16 bzw. zweiten Fluid 17 in Axialrichtung A durchströmt. Wie dargestellt, verjüngt sich der Innenquerschnitt der zweiten Leitung 14, der dem zweiten Fluid 17 zur Verfügung steht, in Axialrichtung A zu der zweiten Öffnung 15. Dies dient dazu, ein in der zweiten Leitung 14 geführtes zweites Fluid 17 vorzubeschleunigen, bevor es an der ersten Öffnung 12 in Axialrichtung A vorbeitritt und dort in Kontakt mit aus der ersten Öffnung 12 in Axialrichtung A ausgestoßenem erstem Fluid 16 kommt. Der Abschnitt mit sich verjüngendem Innenquerschnitt bildet eine Vorbeschleunigungszone 18. Der Bereich stromabwärts der ersten Öffnung 12 kann als Nachbeschleunigungszone 19 bezeichnet werden, da in dieser Bestandteile des zweiten Fluids 17 in den Trägerstrom 20 aus erstem Fluid 17 aufgenommen werden, um von dem Trägerstrom 20 in das Gewebe eines Patienten eingetragen zu werden. Aufgrund des koaxialen Aufbaus bildet der Fluidstrom an zweitem Fluid 17 einen Mantelstrom um den Trägerstrom 20. Dies fördert den umfänglich allseitigen Übertritt von Bestandteilen des zweiten Fluids 17 in das erste Fluid 16. Dazu ist bevorzugt der Normalenvektor der Öffnungsfläche der ersten Öffnung 12, welche Öffnungsfläche von der ersten Leitung 11 umgrenzt wird, parallel zur Axialrichtung A, wobei dies bevorzugt auch für Ausführungsformen erläutert im Zusammenhang mit Figuren 2 bis 4 gilt. Wenn die stirnseitig an der ersten Leitung 11 von der ersten Leitung 11 umgrenzte erste Öffnung 12 in Axialrichtung orientiert ist, fördert dies die gleichmäßige Beladung des Arbeitsstrahls 20 mit Zellen im Wesentlichen gleich nach dem Austreten aus der ersten Leitung 11.

Bevorzugt ist der Abschnitt der Außenwand 22 der zweiten Leitung, zumindest in dem Bereich um die erste Öffnung 12, flexibel, bevorzugt elastisch flexibel. Dies führt zu einem Selbstzentrierungseffekt des flexiblen Abschnitts 22 um die erste Öffnung 12. Dies ist in Figur 1b schematisch dargestellt. Hier ist eine Querschnittsansicht des Instruments 10 gemäß Figur 1a (Querschnittsfläche B eingezeichnet in Figur 1a) dargestellt. Wie ersichtlich ist (nicht maßstabsgetreu) der flexible (bevorzugt elastische) Abschnitt 22 nicht konzentrisch um das Düsenrohr 23 angeordnet, welches das distale Ende der ersten Leitung 11 bildet. Dies kann auf grobe Toleranzen zurückzuführen sein. Wird die zweite Leitung 14 mit zweitem Fluid 17 beaufschlagt, wird der flexible Axialabschnitt 22 der Außenwand, welcher durch die äußere ringförmige Querschnittsfläche repräsentiert wird, von dem zweiten Fluid in Radialrichtung, wie durch die Pfeile P angedeutet, bewegt und dabei zentriert, wie dies in Figur 1b (rechte Abbildung) veranschaulicht ist. Die erste Leitung 11 bleibt dabei in der ursprünglichen Lage. Dies ist entscheidend, denn mit der ersten Leitung 11 kann in einem ersten Schritt ein Kanal in dem Gewebe mittels eines Fluidpulses aus der ersten Leitung nadellos geöffnet werden, in den in einem zweiten Schritt die Bestandteile mittels des Trägerstroms 20 eingebracht werden. Wird der Fluidstrom an zweitem Fluid 17 unterbrochen, kann der ringförmige Außenwandabschnitt 22, sofern er elastisch ist, wieder automatisch in seine Ursprungslage (Figur 1b, linke Abbildung) zurückkehren. Es sind auch Ausführungsformen möglich, welche ohne einen elastischen ringförmigen Außenwandabschnitt der zweiten Leitung auskommen, welcher Selbstzentrierung zeigt.

Figur 2 zeigt eine ausschnittsweise Längsschnittansicht eines weiteren Ausführungsbeispiels eines erfindungsgemäßen Instruments 10. Das Instrument 10 kann für einen offenen, die endoskopischen oder laparoskopischen Eingriff verwendbar sein. Das Instrument 10 kann insbesondere ein Katheterinstrument 10 sein, welches in die Harnröhre einführbar ist, um mit dem Instrument 10 eine Behandlung des Blasenschließmuskels durch Eintragen von Zellen in diesen durchzuführen. Damit kann mittels desselben Instruments 10 durch äußere Schichten des Schließmuskels hindurch in das Muskelgewebe nadellos ein Kanal geschaffen und nadellos Zellen enthaltende Flüssigkeit injiziert werden.

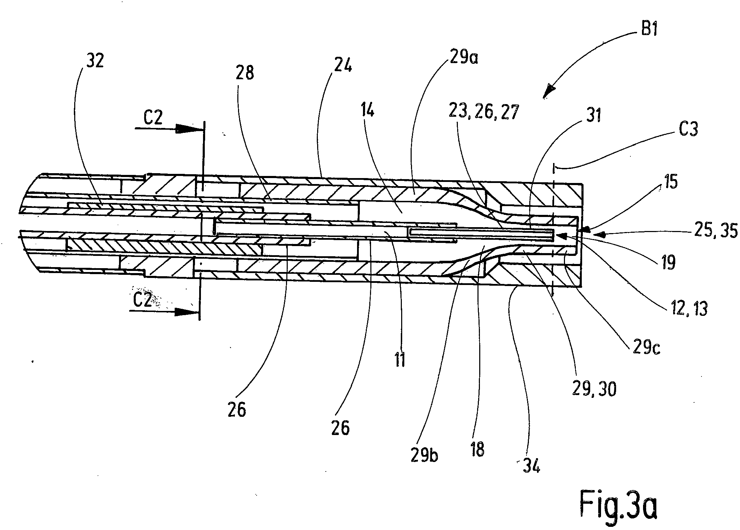

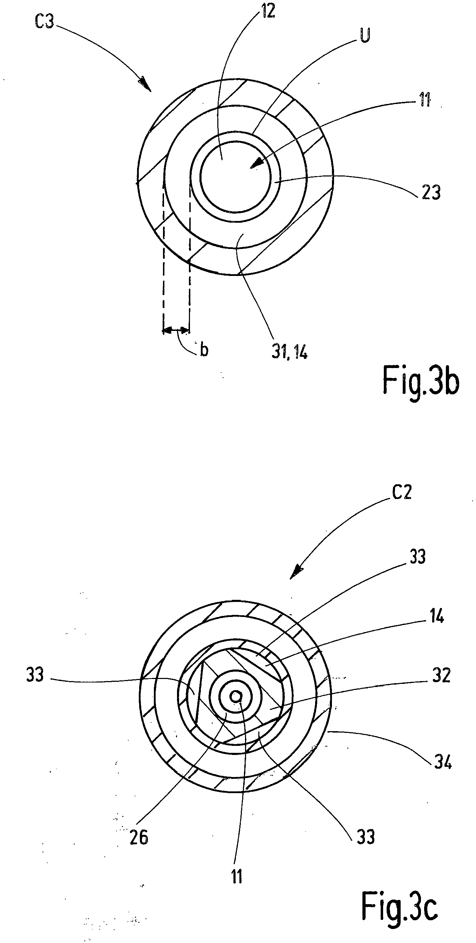

Das Instrument 10 weist einen länglichen Kopf 24 am distalen Ende 25 des Instruments 10 auf. Der Kopf 24 ist unflexibel, starr. Der Abschnitt des Leitungspaares aus den Abschnitten der Leitungen 11, 14 bis zu dem Kopf 24 ist flexibel. In dem Kopf 24 ist eine erste Leitung 11 konzentrisch zu einer zweiten Leitung 14 angeordnet. Wie insbesondere aus Figur 3 hervorgeht, weist die erste Leitung 11 einen Aufbau aus mehreren Leitungsabschnitten auf, welche Innenquerschnittsstufen bilden. Der Innenquerschnitt nimmt in distale bzw. Axialrichtung stufenweise ab. Das Ende der ersten Leitung 11 wird von einem ersten Düsenrohr 23 gebildet. Auch vom proximalen Leitungsabschnitt 26 auf das Düsenrohr 23 nimmt der Innenquerschnitt der ersten Leitung 11 ab, so dass das erste Fluid beim Durchtritt durch die Leitung 11 eine Beschleunigung erfährt. Die Reduzierung des Innenquerschnitts und damit auch des Außenquerschnitts der ersten Leitung 11 in diesem Bereich dient jedoch nicht primär der Beschleunigung des ersten Fluids. Ausführungsformen können auch ohne die Reduktion des Leitungsquerschnitts der ersten Leitung 11 am distalen Ende auskommen. Vielmehr wird durch die Reduktion des Innen- und des Außenquerschnitts eine hohe Stabilität des Düsenrohrs 23 im distalen Bereich und andererseits eine geringere Reduktion des Innenquerschnitts der zweiten Leitung 14 erreicht. Besonders vorteilhaft ist eine Durchmesserreduktion, die kontinuierliche (gleichmäßige) Durchmesserübergänge aufweist (abweichend von der Darstellung gemäß Figur 2 bzw. 3). Ein solches Düsenrohr 24 mit kontinuierlicher Innenquerschnittreduktion in Axialrichtung für die erste Leitung 11 kann beispielsweise einstückig z.B. durch Kneten eines zylindrischen Rohrs (Rundkneten) hergestellt werden, dabei kann auch die Düsenöffnung (erste Öffnung 12) in dem Düsenrohr 23 geschaffen werden.

Die zweite Leitung 14 weist in dem Kopf 24 eine Außenwand auf, welche von zumindest zwei Elementen gebildet ist. Die Innenwand wird von der Außenwand 27 der ersten Leitung 11 gebildet, so dass sich ein ringförmiger Leitungsquerschnitt (Innenquerschnitt) der zweiten Leitung 14 im Kopf 24 des Instruments 10 ergibt. Ein starrer Schaftrohrabschnitt 28 der zweiten Leitung 14 einheitlichen Durchmessers ist an dessen distalem Ende mit einem elastischen Außenwandelement 29 verbunden, welches eine Düse 30 der zweiten Leitung 14 bildet. Das elastische Außenwandelement 29 ist auf das distale Ende des Schaftrohres 28 geschoben. Das elastische Außenwandelement 29 weist einen zylindrischen Abschnitt 29a, einen sich daran anschließenden sich verjüngenden Abschnitt 29b sowie einen weiteren zylindrischen Abschnitt 29c auf. Der zylindrische Abschnitt 29c kann alternativ auch sich, beispielsweise konisch, in Axialrichtung A verjüngend ausgebildet sein. Das Düsenrohr 23 mündet mit einer ersten Öffnung 12 in dem weiteren zylindrischen Abschnitt 29c des elastischen umfänglich geschlossenen Außenwandelements 29. Es ist eine koaxiale Anordnung des ersten Düsenrohrs 23 und der äußeren Düse 30, welche mit dem elastischen Außenwandelement 29 gebildet ist, geschaffen. Die äußere Düse 30 für die Zellsuspension weist einen Leitungsquerschnitt in Form eines Ringspalts 31 auf und ist konzentrisch zu dem Düsenrohr 23 angeordnet, so dass der Ringspalt 31 das Düsenrohr 23 umgibt. Der Leitungsquerschnitt des Ringspalts 31 nimmt in Axialrichtung ab. Fließt nun zweites Fluid, insbesondere zellenhaltige Suspension (Zellsuspension), durch die äußere Düse 30, erfährt das zweite Fluid aufgrund des kleiner werdenden Querschnitts der zweiten Leitung 14 eine Vorbeschleunigung benachbart zum distalen Ende 25 des Instruments 10. Mittels der äußeren Düse 30 ist folglich eine Vorbeschleunigungszone 18 ausgebildet. Bevorzugt ist die Beschleunigung jedoch geringer als die Beschleunigung, die die Arbeitsflüssigkeit beim Durchtritt durch das erste Düsenrohr 23 erfährt. In jedem Fall ist die (mittlere) Austrittsgeschwindigkeit der Zellsuspension nach Düsendurchtritt (auf Höhe (neben) der ersten Öffnung 12) geringer als die (mittlere) Austrittsgeschwindigkeit des Arbeitsstrahls in der ersten Öffnung 12. In besonders bevorzugten Ausführungsformen ist die ringspaltförmige äußere Düse 30 für die Zellsuspension so dimensioniert, dass bei gegebenen Volumenströmen bzw. Durchflüssen einer Arbeitsflüssigkeit als erstes Fluid von 5 bis 55 ml/min und insbesondere von 15-26 ml/min und der Zellsuspension von 1-3 ml/min und insbesondere von 15-26 ml/min die Düsenaustrittsgeschwindigkeit (neben der ersten Öffnung 12) der Zellsuspension etwa 50-90% der Düsenaustrittsgeschwindigkeit des Arbeitsstrahls (in der ersten Öffnung 12) beträgt. Dies gilt für die Phase, in welcher Zellsuspension durch die äußere Düse 30 und Arbeitsflüssigkeit durch die erste Öffnung 12 zeitgleich ausgegeben werden, um mittels des Arbeitsfluids Zellen aus der Zellsuspension in einen Kanal im Gewebe einzutragen. Für das Ausbilden des Kanals mittels eines Pilotstrahls aus Arbeitsfluid kann die Düsenaustrittsgeschwindigkeit des Arbeitsstrahls sogar noch höher sein.

Es hat sich als besonders vorteilhaft erwiesen, wenn der Ringspalt 31 des Abschnitts der zweiten Leitung 14 an der ersten Öffnung 12 einen Innenumfang U von wenigstens 20 mal dem mittleren Zellendurchmesser der Zellen in der Zellsuspension und eine radiale Spaltbreite b von höchstens dem zehnfachen oder sogar höchstens dem fünffachen des mittleren Zellendurchmessers aufweist - bevorzugt mindestens jedoch dem vierfachen des mittleren Zellendurchmessers. Figur 3b zeigt einen Schnitt entlang der Schnittebene C3 eingezeichnet in Figur 3a. In Figur 3b dargestellt ist jedoch nur der Bereich der Schnittebene C3 um das Zentrum der ersten Öffnung 12 radial bis einschließlich zur äußeren Umfangsfläche des zylindrischen Abschnitts 29c des flexiblen Außenwandelements 29. Der Innenumfang U des Ringspalts 31 ist gleich dem Außenumfang des ersten Düsenrohrs 23. Beträgt der Außendurchmesser des ersten Düsenrohrs 23 beispielsweise 0,25 mm und damit der Innenumfang U des Ringspalts etwa 0,79 mm, weist der Ringspalt 31 beispielsweise eine Breite b (radiale Spaltbreite) von 0,1 mm auf, wobei von einer Zellsuspension mit einem mittleren Zelldurchmesser von 25 Mikrometer ausgegangen wird. Dadurch wird einerseits die Beschleunigung der Zellsuspension wie oben beschrieben erreicht. Andererseits haben Versuche gezeigt, dass dadurch der Ringspalt 31 ausreichend groß dimensioniert ist, um bei einem durchschnittlichen Durchmesser der verwendeten Zellen von ca. 25 µm einen hinreichend störungsfreien Durchtritt der Zellen zu ermöglichen. Der Außendurchmesser des Arbeitsflüssigkeitsstrahls (Trägerstrahl) beim Verlassen der ersten Öffnung 12 entspricht bis auf die doppelte Wandstärke des Düsenrohrs 23 dem Innendurchmesser des Ringspalts 31 an der ersten Öffnung 12. Durch Ausbildung des Mantelstrahls 21 an Zellsuspension wird ein Mantelstrahl um den Arbeitsflüssigkeitsstrahl 20 (s. Figur 1) erzeugt, in welchem auf Grund der radialen Abmessung des Ringspalts 29 die Zellen nahe des Arbeitsflüssigkeitsstrahls 20 neben der ersten Öffnung 12 vorbeitreten. So kann eine besonders hohe Aufnahme von Zellen in den Arbeitsflüssigkeitsstrahl 20 gewährleistet werden.

Zwischen dem starren Schaftrohrabschnitt 28 und der ersten Leitung 11 ist ein Zentrierelement 32 angeordnet, wie auch aus Figur 3c hervorgeht. Das Zentrierelement 32 erlaubt einen Fluiddurchtritt durch die zweite Leitung 14, obwohl es in der zweiten Leitung 14 angeordnet ist. Das Zentrierelement 32 sorgt für eine koaxiale Anordnung des Schaftrohres 28 sowie der ersten Leitung 11. Das Zentrierelement 32 weist eine oder mehrere in Axialrichtung offene Ausnehmungen oder Freistellen 33 in Form von beispielsweise Längsnuten auf der Mantelfläche des Zentrierelements 32 auf. Die Zellsuspension gelangt durch die Ausnehmungen 33. Die Ausnehmungen 33 weisen bevorzugt einen einheitlichen Querschnitt auf. Bevorzugt sind die Ausnehmungen gleichmäßig über den Umfang des Zentrierelements 32 verteilt. Das Zentrierelement 32 sorgt letztendlich für eine konzentrische Anordnung des ersten Düsenrohrs 23 für die Arbeitsflüssigkeit 16 und des elastischen Außenwandelements 29 zur Ausbildung des Mantelstrahls 21. Das Zentrierelement 32 stellt, wie im Ausführungsbeispiel gemäß Figur 2 dargestellt, die konzentrische Ausrichtung des ersten Düsenrohrs 23 mit dem elastischen Außenwandelement 29 mittelbar über das Schaftrohr 28 her.

Die ausgewählte elastische Nachgiebigkeit des elastischen Elements, durch Verwenden eines Werkstoffs mit ausgewählter Härte und Form, kann während des Durchtritts von Zellsuspension durch die äußere Düse zu einer Selbstzentrierung und Justage der konvergierenden äußeren Düse, insbesondere des Ringspalts 29, führen, wie dies im Zusammenhang mit dem Ausführungsbeispiel gemäß Figur 1a und insbesondere anhand von Figur 1b erläutert ist. Eine etwa infolge von Fertigungstoleranzen eventuell verbleibende Asymmetrie des Ringspalts 29 wird kompensiert, indem während der Applikation der Zellsuspension über den Zellsuspensionsstrom auf das elastische Außenwandelement 29 eine Kraft ausgeübt wird und dieses dadurch so ausgelenkt wird, dass ein symmetrischer Ringspalt 29 erzeugt wird. Das elastische Außenwandelement 29 besteht vorzugsweise aus einem biokompatiblen und für kurzzeitigen (bis 25 Stunden) Zellkontakt nicht toxischen elastischen Werkstoff, vorzugsweise aus einem Elastomer wie Silikon oder einem thermoplastischen Elastomer. Die Dicke des elastischen Außenwandelements 29 beträgt beispielsweise 0,05 bis 1 mm, vorzugsweise 0,1 bis 0,4 mm. Die Werkstoffhärte liegt im Bereich von 3 Shore (A) bis 70 Shore (A) vorzugsweise im Bereich von 20 Shore (A) und 55 Shore (A).

Das elastische Außenwandelement 29 wird von einer in Axialrichtung drucksteifen Kappe 34 umgeben, welche das distale Ende 25 des Instruments 10 bildet. Die Kappe 34 weist eine Öffnung 35 an der distalen Stirnseite der Kappe 34 auf, gegenüber welcher die Öffnung 15 an der Stirnseite des elastischen Außenwandelements 29 (welche die zweite Öffnung 15 an der Stirnseite der zweiten Leitung 14 ist) proximal entlang der Axialrichtung A zurückversetzt ist. Die Stirnseite der Kappe 34 bildet das distale Ende 25 des Instruments 10. Das Instrument 10 ist mittels der Kappe 34 derart drucksteif eingerichtet, dass es einen Mindestabstand zwischen der distalen Öffnung 35 des Instruments 10, welche auf das Gewebe des Patienten aufdrückbar ist, und der ersten Öffnung 12 und/oder der zweiten Öffnung 15 festlegt, auch wenn auf das distale Ende 25 des Instruments 10 beim Drücken des Instruments 10 gegen das Gewebe eine Kraft ausgeübt wird. Über das Zentrierelement 32 ist auch die Lage der ersten Leitung 11 bezüglich der Kappe 34 festgelegt. Dies hat den Vorteil, dass wenn das Instrument 10 mit der Kappe 34 auf das Gewebe aufgesetzt und mit einem Fluidstoß aus der ersten Leitung 11 ein Kanal in dem Gewebe geöffnet wird, die erste Öffnung 12 über dem so geschaffenen Kanal bleibt, so dass ein folgender Strahl oder Strom aus erstem Fluid 16 mit Bestandteilen des zweiten Fluids 17 sicher in den Kanal trifft.

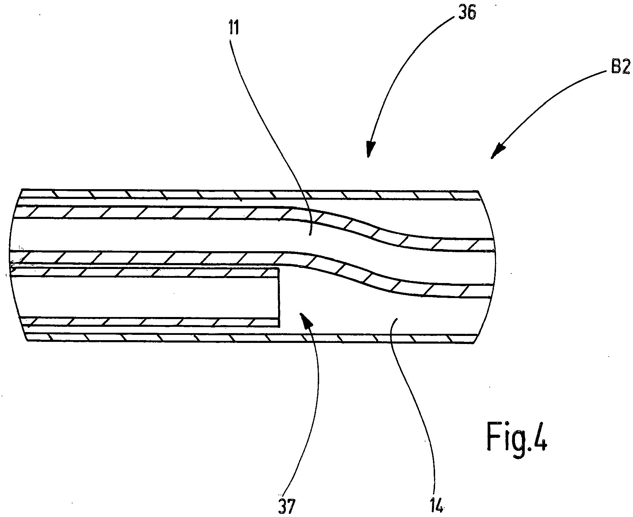

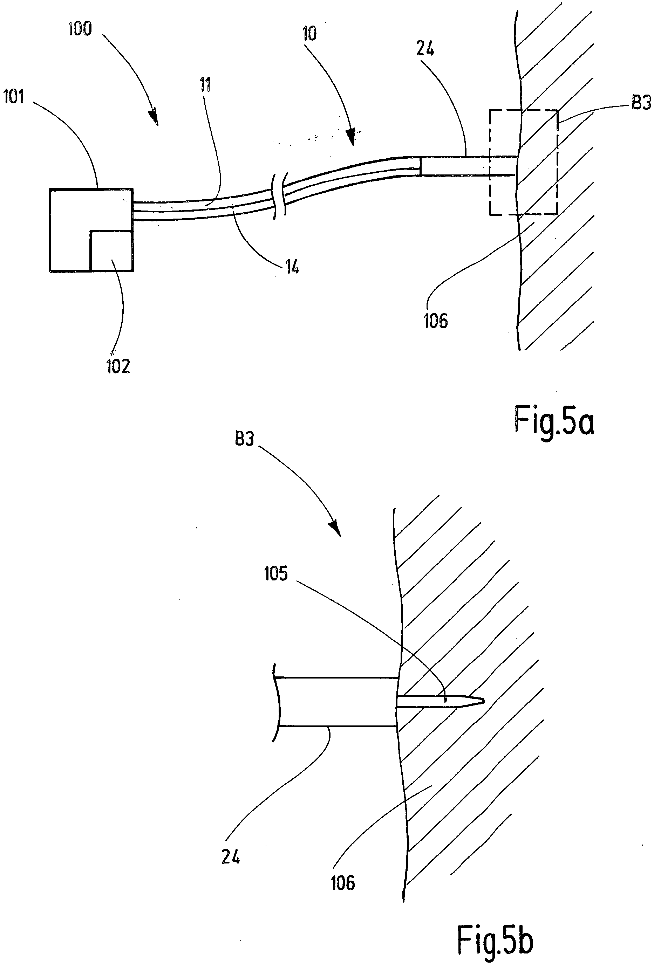

Figur 4 zeigt den Ausschnitt B2 gemäß Figur 2 vergrößert. Wie insbesondere aus den Figuren 2 und 5 beispielhaft hervorgeht, sind die erste Leitung 11 und die zweite Leitung 14 bevorzugt bis benachbart zum distalen Ende 25 des Instruments 10 nebeneinander, insbesondere parallel geführt und anschließend, in Axialrichtung A weiter zum distalen Ende 25, koaxial. Im Ausführungsbeispiel sind die erste Leitung 11 und die zweite Leitung 14 bis in den Kopf 24 nebeneinander angeordnet und, auf eine Übergangsstelle 36 im Kopf 24 folgend, an welcher mittels einer Weichenanordnung 37 von der parallelen Anordnung auf die koaxiale Anordnung gewechselt wird, koaxial. Die Übergangsstelle 36 kann auch stromaufwärts benachbart zum Kopf 24 sein, so dass die erste Leitung 11 und die zweite Leitung 14 bis an den Kopf 24 heran nebeneinander, verschlungen oder parallel, angeordnet und daran in Strömungsrichtung A zum distalen Ende 25 anschließend, stromaufwärts des Kopfes 24 und in dem Kopf 24 koaxial angeordnet sind. Proximal oder stromaufwärts der Übergangsstelle 36 oder proximal des Kopfes 24 können die proximal der Übergangsstelle 36 benachbarten, insbesondere parallelen, erste Leitung 11 und zweite Leitung 14 mittels eines Hüllschlauches 38 zusammengefasst sein. Der Hüllschlauch 38 ist in Figur 2 beispielhaft und mit gestrichelten Linien um die erste Leitung 11 und zweite Leitung 14 veranschaulicht. Der Hüllschlauch 38 kann beispielsweise bis an (wie veranschaulicht), bis in oder bis über den Kopf 24 reichen. Der Übergang von der parallelen auf die konzentrische Kanalanordnung oder Leitungsanordnung kann beispielsweise mittels einer Weichenanordnung 37 geschaffen sein, wie sie in Figur 3 beispielhaft dargestellt ist. Selbstverständlich können die in Figur 2 und Figur 4 getrennt nebeneinander dargestellten ersten und zweiten Leitungen 11, 14 auch durch einen Leitungskörper mit nebeneinander angeordneten separaten, insbesondere parallelen, Kanälen realisiert sein. Ob durch zwei getrennte Leitungen 11, 14 oder zwei Leitungen in Gestalt eines Leitungskörpers enthalten nebeneinander zwei Kanäle, ist die Belastung der Zellen beim Kanaldurchtritt im Bereich der parallelen Kanalanordnung potentiell geringer als bei der koaxialen Anordnung - gleiche Strömungsquerschnitte vorausgesetzt. Denn die Koaxialität des inneren und äußeren Kanals könnte durch viele Zentrierelemente in dem Ringraum zwischen innerer und äußerer Leitung sichergestellt werden - dies würde aber erhöhte Scherkräfte an den Zentrierelementen bedeuten - oder es könnte auf eine Sicherstellung der Koaxialität verzichtet werden, so dass die innere Leitung, insbesondere bei Abwinklung, innen an der äußeren Leitung zum anliegen kommen könnte. Nichtsdestotrotz erreicht man durch die konzentrische Kanalanordnung in dem auf die Übergangsstelle 36 folgenden Bereich die bereits erwähnte bevorzugte Ausbildung einer mantelartigen Einhüllung des Flüssigkeitsstroms 20 aus Zellsuspension, welcher Flüssigkeitsstrom 20 aus der ersten Öffnung 12 ausgestoßen wird. Es wurde gefunden, dass durch den kurzen Bereich zwischen Übergangsstelle 36 und dem distalen Ende 25 des Instruments 10, in welchem die Zellsuspension auf Grund des koaxialen Aufbaus durch einen Ringquerschnitt strömt, eine Schädigung der Zellen beim Durchlaufen der zweiten Leitung 14 im koaxialen Bereich minimiert ist. Es konnte insbesondere gezeigt werden, dass bei einer Länge L des koaxialen Bereichs (von der Übergangsstelle 36 bis zur ersten Öffnung 12) von kleiner oder gleich 40 Millimeter im Wesentlichen keine Beeinträchtigung der Zellvitalität zu verzeichnen ist. Auf die im Zusammenhang mit Figur 3a beschriebene Zentriereinrichtung mittels des Zentrierelements 32 kann beispielsweise verzichtet werden, wenn an einer Stelle, an welcher die erste Leitung 11, wie in Figur 4 dargestellt, ohnehin nahe an der Wand des Kopfes 24 angeordnet ist, die erste Leitung 11 mit der Wand des Kopfes 24, welche stromabwärts der Weichenanordnung 37 (des Übergangs von paralleler Anordnung der ersten Leitung 11 und zweiten Leitung 14 auf koaxiale Anordnung) die zweite Leitung 14 bildet, verschweißt ist.

Figur 5a zeigt stark schematisiert ein Ausführungsbeispiel eines Applikationssystems 100, welches ein erfindungsgemäßes Instrument 10, beispielsweise ein erfindungsgemäßes Instrument 10 gemäß einem der oben erläuterten Ausführungsbeispiele, aufweist. Das System 100 weist zudem eine Versorgungseinrichtung 101 mit einer Steuerung 102 zur Steuerung der Versorgungseinrichtung 101 auf. Die Versorgungseinrichtung 101 weist eine Quelle 103 für erstes Fluid 16 und eine Quelle 104 für zweites Fluid 17 auf.

Die Versorgungseinrichtung 101 kann dazu eingerichtet sein, ein erstes Fluid 16 bereitzustellen, welches beispielsweise eine Flüssigkeit, insbesondere eine Suspension, Lösung, Emulsion oder dergleichen, sein kann. Diese Flüssigkeit kann auch als Arbeitsflüssigkeit bezeichnet werden, denn sie dient in einem ersten Schritt der Schaffung eines Kanals 105 im Gewebe 106, auf welches das Instrument 10 wie in Figur 5b veranschaulicht aufgesetzt sein kann, und in einem zweiten Schritt dem Eintrag von Bestandteilen des zweiten Fluids in den Kanal 105. Zudem stellt die Versorgungseinrichtung 101 ein zweites Fluid 17 bereit, welches Bestandteile aufweist, welche mittels des Instruments 10 bzw. des Systems 100 in dem Kanal 105 im Gewebe 106 des Patienten deponiert werden sollen. Die Bestandteile können beispielsweise Zellen, Zellbestandteile, Arzneimittel, radioaktive Stoffe zum Markieren oder Behandeln oder dergleichen sein. Die Versorgungseinrichtung 101 steht mit der ersten Leitung 11 und der zweiten Leitung 14 in fluider Verbindung.

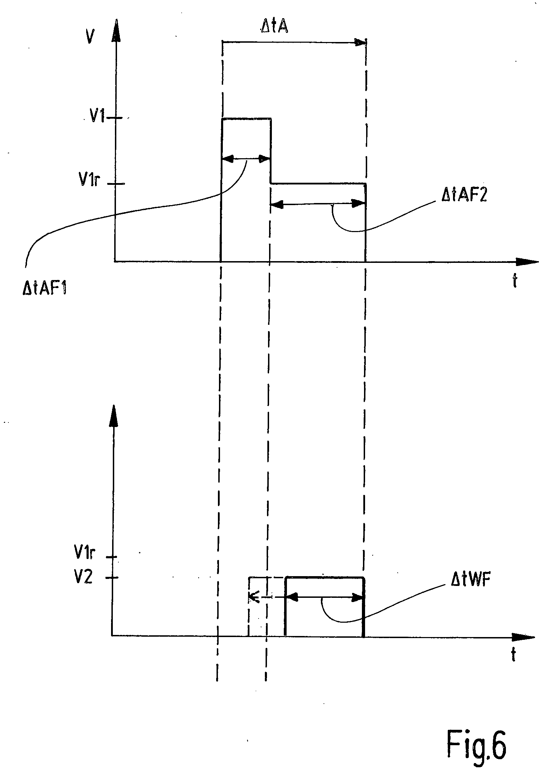

Die Versorgungseinrichtung 101 ist mittels der Steuerung 102 dazu ausgebildet, das erste Fluid 16 und das zweite Fluid 17 in einer Abfolge von Förderintervallen zu fördern. Die Steuerung 102 ist bevorzugt dazu eingerichtet, das Applikationssystem 100 derart zu steuern, dass innerhalb eines Applikationszeitintervalls ΔtA, ein solches ist beispielhaft in Figur 6 veranschaulicht, während eines ersten Arbeitsfluidförderintervalls ΔtAF1 das erste Fluid 16 gefördert wird, so dass dieses an der ersten Öffnung 12 eine erste Intensität, insbesondere erste Geschwindigkeit v1, zum Bilden eines Kanals 105 im Gewebe 106 aufweist. Während eines zweiten Arbeitsfluidförderintervalls ΔtAF2 steuert die Steuerung das Applikationssystem 100 derart, dass das erste Fluid 16 gefördert wird, so dass es an der ersten Öffnung 12 eine reduzierte erste Intensität, insbesondere reduzierte erste Geschwindigkeit v1r, aufweist, die kleiner als die erste Geschwindigkeit v1 ist. Zudem ist die Steuerung 102 dazu eingerichtet, das Applikationssystem 100 zu steuern, das zumindest abschnittsweise während des zweiten Arbeitsfluidförderintervalls ΔtAF2 das zweite Fluid 17 gefördert wird, so dass dieses an der ersten Öffnung 12 eine zweite Geschwindigkeit v2 aufweist, die kleiner als die reduzierte erste Geschwindigkeit v1r ist. Die Ausgabe des zweiten Fluids 17 neben der ersten Öffnung 12 in die Nachbeschleunigungszone 19 in dem Wirkstofffluidförderintervall ΔtWF kann bereits während des ersten Arbeitsfluidförderintervalls ΔtAF1 vor dem Ändern der Intensität synchron mit der Änderung der Intensität in der ersten Öffnung 12 oder nach der Änderung der Intensität, insbesondere ersten Geschwindigkeit v1 in der ersten Öffnung 12 in Axialrichtung A, sein. Die gewünschte zweite Geschwindigkeit v2 des zweiten Fluids 17 stellt sich unter anderem deshalb auf Höhe der ersten Öffnung 12 ein, da dieses durch den sich benachbart zum distalen Ende verjüngenden Ringquerschnitt der zweiten Leitung 14 strömt. Stromaufwärts der äußere Düse strömt das zweite Fluid 17 mit einer niedrigeren Geschwindigkeit, was die Gefahr vermindert, dass Zellen in dem zweiten Fluid 17 beschädigt werden. Das Applikationszeitintervall kann beispielsweise weniger als 1 Sekunde lang sein.

Mit dem erfindungsgemäßen Applikationssystem 100 bzw. dem erfindungsgemäßen Instrument 10 kann beispielsweise wie folgt gearbeitet werden:
Um die Beanspruchung der Zellen während des Applikationsvorgangs zu minimieren und dennoch eine ausreichende Eintragstiefe der Zellen zu erreichen, wird der Applikationsvorgang wie bereits erwähnt bevorzugt mindestens zweistufig durchgeführt. In einer ersten Phase wird dabei der Kanal 105 im Gewebe 106 vorbereitet. Dazu wird in dem ersten Arbeitsfluidförderintervall ΔtAF1 (s. Figur 7) zunächst ein Pilotarbeitsstrahl mit hoher Intensität, insbesondere hoher Düsenaustrittsgeschwindigkeit v1, erzeugt. Dieser Pilotarbeitsstrahl 20 besteht aus der Arbeitsflüssigkeit 16, deren Bestandteile unempfindlich gegenüber mechanischer Belastung, insbesondere Scherbeanspruchung sind. Solche Flüssigkeiten umfassen beispielsweise physiologische Kochsalzlösung, ein Zellkulturmedium, ein gelartiges Benetzungs- oder Hüllmedium für die Zellen des zweiten Fluids zur Erhöhung des mechanischen Schutzes und/oder des Gleitvermögens oder ein Gemisch daraus. Der Kanal 105 wird in dem Gewebe 106 mittels des Pilotarbeitsstrahls 20 geschaffen, indem der Pilotarbeitsstrahl 20 Gewebebestandteile entlang des zu schaffenden Kanals mechanisch verdrängt und/oder zerstört. In einer zweiten Phase wird in dem zweiten Arbeitsfluidförderintervall ΔtAF2 die Intensität des Arbeitsstrahls 20 reduziert, insbesondere die Geschwindigkeit in der ersten Öffnung auf die reduzierte erste Geschwindigkeit v1r vermindert (s. Figur 7). Es kann im Zuge dessen auch die Zusammensetzung des ersten Fluids 16 geändert werden, insbesondere durch Zugabe von Stoffen oder aber durch Wechsel des Arbeitsfluids. Bevorzugt aber ist die Zusammensetzung des aus der ersten Öffnung 12 ausgegebenen Fluids in der ersten und der zweiten Phase gleich. Im Zuge der Reduzierung der Intensität, insbesondere Geschwindigkeit des Arbeitsstrahls 20, wird die Ausgabe des Mantelstrahls 21 aus Zellsuspension aus der zweiten Öffnung 15 gestartet. Wie in dem Schema in Figur 7 unten dargestellt, kann der Start vor der Reduzierung der Intensität des Arbeitsstrahls in dem ersten Arbeitsfluidförderintervall (dies ist in Figur 7 unten mit gestrichelter Linie angedeutet), synchron mit der Reduzierung oder nach der Reduzierung (dies ist in Figur 7 unten mit durchgezogener Linie angedeutet) erfolgen. Das zweite Fluid 17 kann zusätzlich zu den Zellen in der Suspension ein gelartiges Benetzungs- oder Hüllmedium aufweisen. Während des Schaffens des Kanals 105 mittels des Pilotstrahls ist die zweite Leitung 14 vorzugsweise bis in den Bereich in dem Kopf 24, in welchem die erste Leitung 11 und zweite Leitung 14 koaxial verlaufen mit Zellsuspension gefüllt, vorzugsweise nahezu komplett bis zur Höhe (in Axialrichtung gemessen) der ersten Öffnung 12, um eine praktisch verzögerungsfreie Zufuhr der Zellsuspension in den geschaffenen Kanal 105 zu ermöglichen. Dies ist auch eine Folge des erfindungsgemäßen Vorsehens von zwei separaten Leitungen 11, 14 für das erste und das zweite Fluid und des Zusammenführens des ersten Fluids und des zweiten Fluids erst in der Nachbeschleunigungszone 19 am distalen Ende 25 des Instruments 10.

Wenn die Zellsuspension durch die äußere Düse an der ersten Öffnung 12 in Distalrichtung vorbeitritt, umgibt diese den aus der ersten Öffnung 12 der mit dem ersten Düsenrohr 23 gebildeten inneren Düse zentral austretenden Arbeitsstrahl 20 mantelförmig, bevorzugt symmetrisch, ggf. auf Grund der Selbstzentrierung des elastischen Außenwandabschnitts 22 bzw. des Außenwandelements 29. Dadurch wird eine gleichmäßige Beladung der Oberfläche des Arbeitsstrahls 20 mit Zellen bei gleichzeitig weitgehend einheitlicher Strömungsgeschwindigkeit des zweiten Fluids 17 erreicht. Daraus folgt eine vergleichsweise geringe Wechselwirkung zwischen den Zellen untereinander, was eine geringe Zellbelastung begünstigt. Mittels des Instruments 10 werden die Arbeitsflüssigkeit 16 und die Zellsuspension derart ausgegeben, dass der Arbeitsflüssigkeitsstrahl, stromabwärts der ersten Öffnung 12, die den Arbeitsstrahl 20 mantelförmig umgebende Zellsuspension 17 radial nach innen zum Arbeitsstrahl 20 ansaugt. Dies wird auf den Bernoulli-Effekt zurückgeführt, welcher auch bei einer Strahlpumpe Anwendung findet und steht im Gegensatz zu einer einfachen Mischung rein durch Überlagerung zweier Fluidstrahle. Wenn die Arbeitsflüssigkeit 16 und die Zellsuspension 17 distal des Düsenrohres 23 miteinander in Berührung kommen (insbesondere, wie oben beschrieben durch Ansaugen des Mantelstrahls 21 durch den Arbeitsfluidstrahl 20), werden die Zellen durch den Arbeitsstrahl auf- und mitgenommen und beschleunigt, bis die Geschwindigkeit der Zellen der Geschwindigkeit des Arbeitsstrahls 20 entspricht. Die geringe Beschleunigung der Zellsuspension 17 in der Vorbeschleunigungszone 18 in der äußeren Düse hat den Vorteil, dass die Zellen an den radial innenliegenden und der radial außenliegenden, den Kanal in der zweiten Leitung 14 begrenzenden Wandflächen der äußeren Düse nur eine geringe Scherbeanspruchung erfahren, dass aber andererseits die Relativgeschwindigkeit zwischen den Zellen und dem Arbeitsfluid 20 beim Erstkontakt des ersten und zweiten Fluids 16, 17 in der Nachbeschleunigungszone 19 geringer als die Strömungsgeschwindigkeit des Arbeitsfluids 16 ist. Die bestehende Geschwindigkeitsdifferenz der beiden Flüssigkeiten 16, 17 in dem Bereich, an welchem diese zum ersten Mal miteinander in Kontakt kommen, führt zwar ebenfalls zur Erzeugung von Scherbeanspruchung der Zellen, insgesamt wird aber eine geringe Zellbeanspruchung und damit eine hohe Überlebensrate der Zellen bei der Injektion in den Kanal 105 im Gewebe 106 erreicht. Die vorliegende Erfindung begünstigt, wie im Zusammenhang mit Figur 3 erläutert, durch Wechsel benachbart zum distalen Ende des Instruments 10 von nebeneinander, insbesondere parallel, verlaufenden Kanälen auf koaxiale Kanäle der ersten und zweiten Leitung 11, 14 das Überleben der Zellen zusätzlich. Auch dadurch, dass erfindungsgemäß ein außenmischender Ansatz verfolgt wird, indem das Instrument 10 über Leitungen 11, 14 für das erste Fluid 16 und das zellenenthaltene zweite Fluid 17 enthält und das Zumischen der Zellsuspension 17 bzw. der Zellen in den Arbeitsstrahl 20 erst nach dem Durchtritt durch das erste Düsenrohr 23 erfolgt, wird eine hohe Überlebensrate der Zellen begünstigt. Eine wie in Figur 1 oder 2 dargestellte zweite Düse, gebildet von der Außenfläche des Düsenrohrs 23 und der Innenfläche des Außenwandabschnitts 22 bzw. Außenwandelements 29 ist durch die kontinuierliche Verjüngung zumindest des Innendurchmessers des elastischen Außenwandelements 29 besonders scherströmungsarm und damit eine hohe Überlebensrate der Zellen begünstigend.

Die Mitnahme der Zellsuspension 17 bzw. der Zellbestandteile durch den Arbeitsstrahl 20 wird begünstigt, wenn wie in den Figuren 1 und 2 beispielhaft dargestellt, das distale Ende des ersten Düsenrohrs 23 gegenüber dem distalen Ende des elastischen Außenwandelements 29 zurückgesetzt ist. Die Nachbeschleunigungszone 19, in welcher eine Mischung stattfindet, wird damit axialabschnittsweise von dem elastischen Außenwandelement 29, genauer dem weiteren zylindrischen Abschnitt 29c, umfasst. Die Gestalt des Außenwandelements 29 und die Anordnung von Außenwandelement 29 und Düsenrohr 23 haben den Vorteil, dass der Mantelstrahl 21, insbesondere durch den Abschnitt 29c, stabil radial eng an das Düsenrohr 23 und letztendlich den zentralen Arbeitsstrahl 20 herangeführt wird, und zwar bevorzugt im Wesentlichen so, dass Mantelstrahl 21 und Arbeitsstrahl 20 überwiegend dieselben Richtungskomponenten haben. Das distale Ende des Düsenrohrs 23, welches die erste Öffnung 12 bildet, kann gegenüber dem distalen Ende des elastischen Außenwandabschnitts 29, welches die zweite Öffnung 15 bildet, beispielsweise 0,5 bis 3 mm zurückversetzt sein. In einem besonders bevorzugten Ausführungsbeispiel ist das Düsenrohr 23 um 1 mm zurückgesetzt. Durch den Versatz der ersten Öffnung 12 an der distalen Stirnseite 13 der ersten Leitung zu dem distalen Ende 25 des Instruments 10 wird eine Mindestlänge der Nachbeschleunigungszone 19, in welcher auch das Aufnehmen von Bestandteilen des zweiten Fluids 17 in das erste Fluid 16 erfolgt, innerhalb des Instruments 10 festgelegt. Die Nachbeschleunigungszone 19 ist auch dann weitgehend frei, wenn das distale Ende 25 des Instruments 10 auf Gewebe 106 aufgedrückt wird.

Wenn das distale Ende 25 des Instruments 10 leicht auf die Gewebeoberfläche gedrückt wird, kann erreicht werden, dass sich die Lage des distalen Endes 25 des Instruments 10 und insbesondere des ersten Düsenrohrs 23 während der Schaffung des Kanals 105 und der Applikation der Zellen nicht ändert. Dazu verfügt die beispielhafte Ausführungsform des Instruments 10 gemäß Figur 2 über eine Kappe 34 aus einem in axiale Richtung A drucksteifen und biegesteifen Material, welche verhindert, dass Gewebe beim Aufsetzen des Instruments zu dem elastischen Außenwandelement 29 gelangt und dieses deformiert.

Ein Applikationsvorgang kann zusätzlich zur nadellosen Erzeugung des Injektionskanals 105 in dem Gewebe 106 im Zielgebiet mit dem Instrument 10 und der Injektion der Zellsuspension in der festgelegten Dosierung bis in den Kanal 105. Der Applikationsvorgang kann zusätzlich auch noch die anschließende Abdichtung des Injektionskanals 105 beinhalten, beispielsweise mit einem im Anschluss an die Zellsuspension 17 über die zweite Leitung 14 in den Arbeitsflüssigkeitsstrahl 20 beigemischten Abdichtmedium, welches höher viskos als die Zellsuspension und gelartig sein kann.

Um eine hohe Wirksamkeit von einer in Gewebe eines Patienten eingebrachten Substanz zu gewährleisten, beispielsweise um eine hohe Intaktheit von in Gewebe eines Patienten eingebrachten Zellen zu gewährleisten, ist erfindungsgemäß ein Instrument (10) mit einer ersten Leitung (11) zum Ausgeben eines ersten Fluides (16) aus einer ersten Öffnung (12) der ersten Leitung (11) in Axialrichtung (A) in eine Nachbeschleunigungszone (19) und mit einer zweiten Leitung (14) zum Leiten von zweitem Fluid (17) in die Nachbeschleunigungszone (19) in Axialrichtung (A), so dass Wirkstoffbestandteile, beispielsweise Zellen, des zweiten Fluids (17) in Folge des Strömens in die Nachbeschleunigungszone (19) von dem in die Nachbeschleunigungszone (19) eintretenden ersten Fluid (16) in Axialrichtung (A) nachbeschleunigt werden. Es wird ein koaxialer Aufbau der ersten und zweiten Leitungen (11, 14) bevorzugt, auf Grund dessen um die erste Öffnung (12) ein in Axialrichtung (A) stromabwärts reichender Mantelstrahl (21) aus zweitem Fluid (17) um den zentralen Arbeitsstrahl (20) erzeugt werden kann. Zudem ist ein Kopf (24) für ein erfindungsgemäßes Instrument (10) und ein Applikationssystem (100) mit einem erfindungsgemäßen Instrument (10) geschaffen. In beispielhaften, nicht dargestellten Ausführungsformen des Instruments kann das Instrument mehrere, d.h. wenigstens zwei, zweite Leitungen aufweisen. Mit den mehreren zweiten Leitungen können abwechselnd und/oder, zumindest teilweise, zugleich gleiche oder unterschiedliche zweite Fluide in die Nachbeschleunigungszone geleitet werden, so dass Bestandteile der zweiten Fluide in Folge des Strömens in die Nachbeschleunigungszone von dem in die Nachbeschleunigungszone eintretenden ersten Fluid in Axialrichtung nachbeschleunigt werden. Die zweiten Fluide können beispielsweise unterschiedliche Substanzen sein oder unterschiedliche Substanzen, insbesondere Wirkstoffe, enthalten. Alternativ oder zusätzlich können Ausführungsformen des erfindungsgemäßen Instruments auch mehrere erste Leitungen, z.B. wenigstens zwei, erste Leitungen aufweisen, welche dazu eingerichtet sind gleiche oder unterschiedliche erste Fluide in eine Nachbeschleunigungszone auszugeben, um ein oder mehrere zweite Fluide nachzubeschleunigen.

**Bezugszeichenliste:**

| | |
|---|---|
| 10 | Instrument |
| 11 | erste Leitung |
| 12 | erste Öffnung |
| 13 | Stirnseite |
| 14 | zweite Leitung |
| 15 | zweite Öffnung |
| 16 | erstes Fluid |
| 17 | zweites Fluid |
| 18 | Vorbeschleunigungszone |
| 19 | Nachbeschleunigungszone |
| 20 | Arbeitsstrahl/Trägerstrahl |
| 21 | Mantelstrahl |
| 22 | Außenwandabschnitt |
| 23 | Düsenrohr |
| 24 | Kopf |
| 25 | distales Ende des Instruments |
| 26 | Leitungsabschnitte |
| 27 | Außenwand der ersten Leitung/Innenwand der zweiten Leitung |
| 28 | Schaftrohrabschnitt |
| 29 | Außenwandelement |
| 29a | zylindrischer Abschnitt |
| 29b | verjüngender Abschnitt |
| 29c | weiterer Zylindrischer Abschnitt |
| 30 | äußere Düse |
| 31 | Ringspalt |
| 32 | Zentrierelement |
| 33 | Ausnehmung/Freistelle |
| 34 | Kappe |
| 35 | Öffnung |
| 36 | Übergangsstelle |
| 37 | Weichenanordnung |
| 38 | Hüllschlauch |
| 100 | Applikationssystem |
| 101 | Versorgungseinrichtung |
| 102 | Steuerung |
| 103 | Quelle |
| 104 | Quelle |
| 105 | Kanal |
| 106 | Gewebe |
| P | Pfeil |
| A | Axialrichtung |
| U | Innenumfang |
| b | Spaltbreite |
| B1, B2, B3 | Ausschnitte |
| C1, C2, C3 | Querschnittsflächen |
| L | Länge |
| ΔtA | Applikationszeitintervall |
| ΔtAF1 | erstes Arbeitsfluidförderintervall |
| ΔtAF2 | zweites Arbeitsfluidförderintervall |
| ΔtWF | Wirkstofffluidförderintervall |
| v1 | erste Geschwindigkeit |
| v1r | reduzierte erste Geschwindigkeit |
| v2 | zweite Geschwindigkeit |

## Patentansprüche

1. Instrument (10) mit einer ersten Leitung (11) zum Ausgeben eines ersten Fluids (16) aus einer ersten Öffnung (12) der ersten Leitung (11) in Axialrichtung (A) in eine Nachbeschleunigungszone (19),
mit einer zweiten Leitung (14) zum Leiten von zweitem Fluid (17) in die Nachbeschleunigungszone (19) in Axialrichtung (A), so dass Bestandteile des zweiten Fluids (17) in Folge des Strömens in die Nachbeschleunigungszone (19) von dem in die Nachbeschleunigungszone (19) eintretenden ersten Fluid (16) in Axialrichtung (A) nachbeschleunigt werden.

2. Instrument (10) nach Anspruch 1 mit einer Vorbeschleunigungszone (18) der zweiten Leitung (14) stromaufwärts der Nachbeschleunigungszone (19), wobei das Instrument (10) dazu eingerichtet ist, das zweite Fluid (17) in der Vorbeschleunigungszone (18) in Axialrichtung (A) vorzubeschleunigen, wobei die erste Öffnung (12) an der Nachbeschleunigungszone (19) angeordnet ist, so dass das aus der ersten Öffnung (12) ausgegebene erste Fluid (16) das vorbeschleunigte zweite Fluid (17) in Axialrichtung (A) nachbeschleunigt.

3. Instrument (10) nach Anspruch 2, wobei die Vorbeschleunigungszone (18) von einem Düsenabschnitt der zweiten Leitung (14) gebildet ist, in welchem sich der Innenquerschnitt in Strömungsrichtung verjüngt.

4. Instrument (10) nach Anspruch 3, wobei sich der Innenquerschnitt des Düsenabschnitts in zumindest einem Unterabschnitt des Düsenabschnitts kontinuierlich verjüngt.

5. Instrument (10) nach einem der vorstehenden Ansprüche, wobei die zweite Leitung (14) die erste Leitung (11) an der ersten Öffnung (12) konzentrisch umgibt, um einen ringförmigen Mantelstrom (21) an zweitem Fluid (17) um das aus der ersten Öffnung (12) austretende erste Fluid (16) zu bilden.

6. Instrument (10) nach einem der vorstehenden Ansprüche, wobei die zweite Leitung (14) dazu eingerichtet ist, den Mantelstrom (21) radial eng an die erste Leitung (11) zu führen, so dass der Arbeitsstrahlstrahl (20), welcher aus der ersten Öffnung (12) austritt, Bestandteile des Mantelstroms (21) in den Arbeitsstrahl (20) einsaugt.

7. Instrument (10) nach einem der vorstehenden Ansprüche, wobei die Außenwand der zweiten Leitung (14) in einem Abschnitt (22, 29) um die erste Öffnung (12) elastisch ist.

8. Instrument (10) nach einem der vorstehenden Ansprüche, wobei der Abschnitt (22) von einem elastischen Element (29) gebildet ist, welches Element (29) die Außenwand der zweiten Leitung (14) im Düsenabschnitt bildet.

9. Instrument (10) nach einem der vorstehenden Ansprüche, wobei das elastische Element (29) stromaufwärts mit einem Rohrabschnitt (28) der zweiten Leitung (14) verbunden ist, wobei radial zwischen dem Rohrabschnitt (28) und der ersten Leitung (11) eine Zentriereinrichtung (32) wirksam ist.

10. Instrument (10) nach einem der vorstehenden Ansprüche, wobei der Rohrabschnitt (28) der zweiten Leitung (14) einen Rohrabschnitt (26) der ersten Leitung (11) umgibt, wobei die Zentriereinrichtung (32) radial zwischen dem Rohrabschnitt (28) der zweiten Leitung (14) und dem Rohrabschnitt (26) der ersten Leitung (11) angeordnet ist, um den Rohrabschnitt (26) der ersten Leitung (11) und den Rohrabschnitt (28) der zweiten Leitung (14) konzentrisch auszurichten.

11. Instrument (10) nach einem der vorstehenden Ansprüche, wobei die zweite Leitung (14) stromabwärts der ersten Öffnung (12) und/oder stromaufwärts der distalen Öffnung (35) des Instruments (10) endet.

12. Instrument (10) nach einem der vorstehenden Ansprüche, wobei das Instrument (10) dazu eingerichtet ist, das erste Fluid (16) und das zweite Fluid (17) bis benachbart zum distalen Ende (25) des Instruments (10) heran nebeneinander in nebeneinander angeordneten Leitungsabschnitten der ersten und zweiten Leitung (11, 14) zu führen und anschließend weiter in Richtung distales Ende in koaxialen Leitungsabschnitten der ersten und der zweiten Leitung (11, 14) zu führen.

13. Instrument (10) nach einem der vorstehenden Ansprüche, wobei die Vorbeschleunigungszone (18) und/oder die Nachbeschleunigungszone (19) in einem Kopf (24) des Instruments (10) angeordnet sind.

14. Instrumentenkopf (24) für ein Instrument (10) gemäß Anspruch 13.

15. Applikationssystem (100) mit:
einem Instrument (10) nach einem der vorstehenden Ansprüche, einer Versorgungseinrichtung (101), die mit der ersten Leitung (11) und der zweiten Leitung (14) in Fluidverbindung steht und dazu ausgebildet ist, das erste Fluid (16) und das zweite Fluid (17) in einer Abfolge von Förderintervallen (ΔtAF1, ΔtAF2, ΔtWF) zu fördern.

16. Applikationssystem (100) nach Anspruch 15, wobei die Versorgungseinrichtung (101) eine Steuerung (102) aufweist, welche das Applikationssystem (100) derart steuert, dass innerhalb eines Applikationszeitintervalls (ΔtA), während eines ersten Arbeitsfluidförderintervalls (ΔtAF1) das erste Fluid gefördert wird, so dass dieses an der ersten Öffnung (12) eine erste Geschwindigkeit (v1) zum Bilden eines Kanals (105) im Gewebe (106) aufweist, während eines zweiten Arbeitsfluidförderintervalls (ΔtAF2) das erste Fluid gefördert wird, so dass es an der ersten Öffnung (12) eine reduzierte erste Geschwindigkeit (v1r) aufweist, die kleiner als die erste Geschwindigkeit (v1) ist, und dass zumindest abschnittsweise während des zweiten Arbeitsfluidförderintervalls (ΔtAF2) das zweite Fluid (17) gefördert wird, so dass dieses an der ersten Öffnung (12) eine zweiten Geschwindigkeit (v2) aufweist, die kleiner als die reduzierte erste Geschwindigkeit (v1r) ist.

17. Verfahren zum Applizieren von Wirkstoff, insbesondere Zellen, in Gewebe (106), wobei
- mittels eines Arbeitsstrahls (20) aus erstem Fluid (16) ausgestoßen aus einer ersten Leitung (11) aus einer ersten Öffnung (12) eines Instruments (10) nadellos ein Kanal (105) in dem Gewebe (106) geöffnet wird, und
- parallel oder koaxial zum Arbeitsstrahl (20) ein Strahl (21) aus zweitem Fluid (17) aus einer zweiten Leitung (14) des Instruments (10) ausgegeben wird, welches zweite Fluid (17) Bestandteile aufweist, welche von dem Arbeitsstrahl (20) aufgenommen werden, um diese in den Kanal (105) zu tragen.
